# EUROPEAN PATENT SPECIFICATION

(11) **EP 2 691 136 B1**
(45) Date of publication and mention of the grant of the patent: **13.07.2016**
(21) Application number: 12713372.6
(22) Date of filing: 28.03.2012
(51) Int. Cl.: A61M 16/00

(54) **METHODS TO TRANSITION ADJACENT TO OR IN CONJUNCTION WITH A SECONDARY AIRWAY PRESSURE THERAPY**
ÜBERGANGSVERFAHREN NEBEN ODER IN VERBINDUNG MIT EINER SEKUNDÄREN ATEMWEGSDRUCKTHERAPIE
PROCÉDÉS DE TRANSITION ADJACENTE OU EN ASSOCIATION AVEC UNE THÉRAPIE DE PRESSION DES VOIES AÉRIENNES SECONDAIRE

(30) Priority: 30.03.2011 US 201161469311 P
(43) Date of publication of application: 05.02.2014
(73) Proprietor: Koninklijke Philips N.V., 5656 AE Eindhoven (NL)
(72) Inventor: SIBENALLER, Sara, Marie, NL-5656 AE Eindhoven (NL); LAURA LAPOINT, Manuel, NL-5656 AE Eindhoven (NL); BALOA WELZIEN, Leonardo, Alberto, NL-5656 AE Eindhoven (NL)
(74) Representative: Steffen, Thomas
(86) International application number: PCT/IB2012/051469
(87) International publication number: WO 2012/131586

(56) References cited:
- WO-A1-2010/106451
- WO-A1-2010/116276
- WO-A1-2010/133986
- WO-A1-2011/004275
- US-A1- 2008 269 629

## Description

The present disclosure pertains to providing pressure support therapy to a subject, and in particular, providing a transition between different modes of pressure support therapy.

Pressure support devices may provide a primary therapy in the form of CPAP, CFlex, BiPAP, Biflex, Aflex, Ventilation and others. In some embodiments, the pressure support devices may also provide a second therapy in the form of, for example, paced breathing. A pressure support device of this kind is known from WO 2010/106451 A1. WO 2010/106451 A1 discloses a system to adjust the tidal volume of the breathing of a subject. However, the change from the primary therapy to the secondary therapy may be sudden and may cause discomfort to the subject. Thus, there is a need for a pressure support system that provides a comfortable, gradual transition between the different therapies.

According to an aspect of the present disclosure, a pressure support system according to claim 1 is provided which includes a pressure generator configured to generate a pressurized flow of breathable gas for delivery to the airway of a subject and a processor configured to execute computer program modules. The computer program modules includes a first mode module configured to control the pressure generator in accordance with a first mode of therapy wherein breathing cues are provided to the subject in accordance with a breathing regime using the pressurized flow of breathable gas. The breathing cues prompt the subject to consciously alter one or more breathing parameters of respiration. The computer modules also include a second mode module configured to control the pressure generator in accordance with a second mode of therapy wherein the flow of breathable gas is provided to the airway of the subject at a selected pressure or selectively variable pressures. The computer modules further include a transition module configured to transition between the first mode of therapy and a second mode of therapy wherein a pressure level of the gas in the pressurized flow of breathable gas is adjusted in response to a trigger event. The transition provided by the transition module between the first mode of therapy and the second mode of therapy includes a gradual adjustment of the a pressure level of the gas in the pressurized flow of breathable gas.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.
FIG. 1 illustrates a system provide pressure support to a subject, in accordance with one or more embodiments of the invention;
FIG. 2 illustrates a user interface configured to provide information to a subject related to breathing cues being provided to the subject according to a secondary therapy, according to one or more embodiments of the invention;
FIG. 3 illustrates a user interface configured to provide information to a subject related to breathing cues being provided to the subject in accordance with a secondary therapy, according to one or more embodiments of the invention;
Fig. 4 illustrates a user interface configured to provide information to a subject related to a primary therapy and/or the secondary therapy, according to one or more embodiments of the invention;
Fig. 5 illustrates a user interface configured to provide information to a subject related to a primary therapy and/or the secondary therapy, according to one or more embodiments of the invention;
Fig. 6 illustrates a user interface configured to provide information to a subject related to a primary therapy and/or the secondary therapy, according to one or more embodiments of the invention;
Fig. 7a illustrates an order in which primary and secondary therapies and a transition between the primary and secondary therapies are provided to subject, according to one or more embodiments of the invention;
Fig. 7b illustrates another order in which primary and secondary therapies and a transition between the primary and secondary therapies are provided to subject, according to one or more embodiments of the invention;
Fig. 8a illustrates a method of providing primary and secondary therapy to a subject and a transition between the primary and secondary therapies, according to one or more embodiments of the invention; and
Fig. 8b illustrates another method of providing primary and secondary therapies and a transition between the primary and secondary therapies to a subject, according to one or more embodiments of the invention.

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

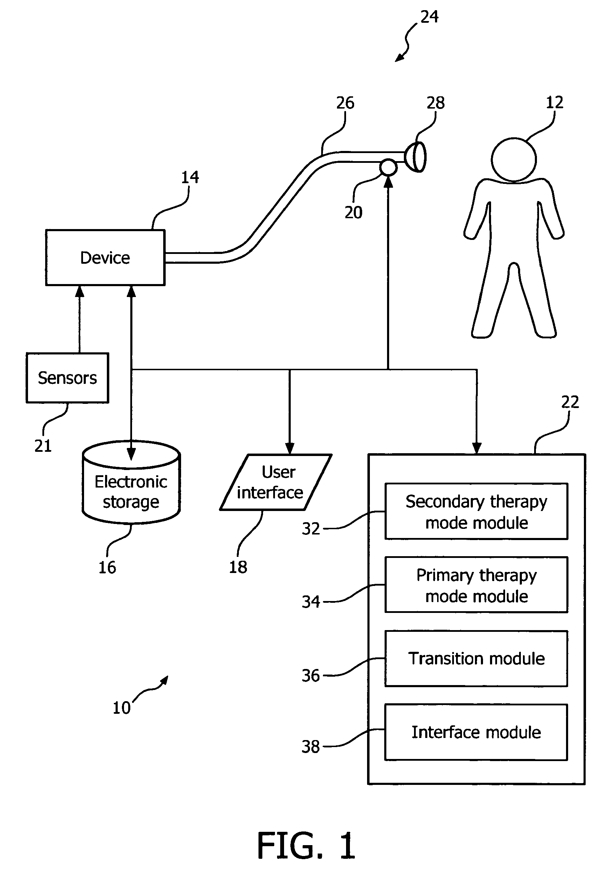

FIG. 1 schematically illustrates an exemplary embodiment of a system 10 configured to deliver a pressurized flow of breathable gas to an airway of a subject 12.

In one embodiment, system 10 may include a pressure generator device 14, pressure controller 15, electronic storage 16, a user interface 18, one or more gas parameter sensors 20, one or more physiological sensors 21, a processor 22, and/or other components.

In one embodiment, device 14 includes a positive pressure support device. A positive pressure support device is well-known and is disclosed, for example, in U.S. Patent 6,105,575, hereby incorporated by reference in its entirety. In this embodiment, device 14 is configured to deliver a pressurized flow of breathable gas to the airway of subject 12.

Device 14 may be configured to generate the pressurized flow of breathable gas according to one or more modes of therapy, such as for example, a primary therapy and a secondary therapy. A non-limiting example of a primary therapy is Continuous Positive Airway Pressure (CPAP). CPAP has been used for many years and has proven to be helpful in promoting regular breathing. Another mode for generating the pressurized flow of breathable gas is Inspiratory Positive Air Pressure

(IPAP). One example of the IPAP mode is bi-level positive air pressure (BiPAP). In BiPAP, two levels of positive air pressure (HI and LO) are supplied to a patient. Other primary therapies are contemplated, such as, for example, CFlex, Biflex, AFlex, and Ventilation. In some embodiments, during primary therapy, continuous positive pressure may be supplied to the airway of subject 12 to keep subject 12's airway open during sleep.

With regard to the BiPAP mode generally, the timing of the HI and LO levels of pressure are controlled such that the HI level of positive air pressure is delivered to subject 12 during inhalation and the LO level of pressure is delivered to subject 12 during exhalation. In conventional positive pressure support devices, the timing of the HI and LO levels of pressure is coordinated to coincide with the breathing of subject 12 based on detection of gas parameters that indicate whether a user is currently inhaling or exhaling. During secondary therapy, the timing of the HI and LO segments of BiPAP may generate breathing cues to prompt subject 12 in changing her breathing rate. In some embodiments, the breathing cues may be provided using HI and LO levels as described in U.S. Patent No. 7,556,038. In some embodiments, the breathing rate may be controlled by adjusting the tidal volume (air breathed per breath) delivered via controlled, progressive pressurization during inspiration and controlled depressurization during expiration.

It should be appreciated that device 14 may also deliver gases other than room air. For example, device 14 can also be oxygen delivery devices, anesthesia devices, fresh air respirators, and respiratory (or other) drug delivery devices in other embodiments. It should also be appreciated that the gas may be positive pressure, negative pressure, or atmospheric.

In some embodiments, system 10 may be configured to provide the secondary therapy that is different from the primary therapy. In one embodiment, system 10 adjusts one or more gas parameters of the gas in the pressurized flow of breathable gas to provide breathing cues to subject 12 that encourage subject 12 to consciously adjust respiration such that the one or more breathing parameters are altered to conform to a breathing regime. This type of therapy may be referred to as "paced breathing." System 10 may be further configured to provide information to subject 12 related to the breathing cues being delivered (or about to be delivered) by system 10 via the pressurized flow of breathable gas. This information may be provided to the user auditorily, visually, tactily, and/or via some other sensory feedback. The information related to the breathing cues may train subject 12 to understand the breathing cues delivered through the pressurized flow of breathable gas, to assume one or more therapeutic body positions, and/or serve other purposes. In other embodiments, the secondary therapy may include providing a level of pressure to subject 12 that is lower than the pressure provided during primary therapy. Alternatively, the secondary therapy may include providing a level of pressure to subject 12 that is higher than the pressure provided during primary therapy.

The pressurized flow of breathable gas is delivered to the airway of subject 12 via a subject interface 24 during the primary and secondary therapies. Subject interface 24 is configured to communicate the pressurized flow of breathable gas generated by device 14 to the airway of subject 12. As such, subject interface 24 includes a conduit 26 and an interface appliance 28. Conduit conveys the pressurized flow of breathable gas to interface appliance 28, and interface appliance 28 delivers the pressurized flow of breathable gas to the airway of subject 12. Some examples of interface appliance 28 may include, for example, an endotracheal tube, a nasal cannula, a tracheotomy tube, a nasal mask, a nasal/oral mask, a full face mask, a total face mask, or other interface appliances that communication a flow of gas with an airway of a subject. The present invention is not limited to these examples, and contemplates delivery of the pressurized flow of breathable gas to subject 12 using any subject interface.

Pressure controller 15 is operative to control the pressure of breathing gas delivered to the airway of subject 12. Pressure controller 15 may be located downstream of device 14. In one embodiment, pressure controller 15 takes the form of an adjustable valve that provides a flow path that is open to the ambient atmosphere via a restricted opening, the valve being adjustable to maintain a constant pressure drop across the opening for all flow rates.

In one embodiment, electronic storage 16 comprises electronic storage media that electronically stores information. The electronically storage media of electronic storage 16 may include one or both of system storage that is provided integrally (i.e., substantially non-removable) with system 10 and/or removable storage that is removably connectable to system 10 via, for example, a port (e.g., a USB port, a firewire port, etc.) or a drive (e.g., a disk drive, etc.). Electronic storage 16 may include one or more of optically readable storage media (e.g., optical disks, etc.), magnetically readable storage media (e.g., magnetic tape, magnetic hard drive, floppy drive, etc.), electrical charge-based storage media (e.g., EEPROM, RAM, etc.), solid-state storage media (e.g., flash drive, etc.), and/or other electronically readable storage media. Electronic storage 16 may store software algorithms, information determined by processor 22, information received via user interface 18, and/or other information that enables system 10 to function properly. Electronic storage 16 may be (in whole or in part) a separate component within system 10, or electronic storage 16 may be provided (in whole or in part) integrally with one or more other components of system 10 (e.g., device 14, user interface 18, processor 22, etc.).

In embodiments that include user interface 18, user interface 18 is configured to provide an interface between system 10 and subject 12 through which subject 12 may provide information to and receive information from system 10. This enables data, results, and/or instructions and any other communicable items, collectively referred to as "information," to be communicated between subject 12 and any combination of one or more of device 14, electronic storage 16, and/or processor 22. Examples of interface devices suitable for inclusion in user interface 18 include a keypad, buttons, switches, a keyboard, knobs, levers, a display screen, a touch screen, speakers, a microphone, an indicator light, an audible alarm, a printer, and/or other interface devices. In one embodiment, user interface 18 includes a plurality of separate interfaces. In one embodiment, user interface 18 includes at least one interface that is provided integrally with device 14.

It is to be understood that other communication techniques, either hardwired or wireless, are also contemplated by the present invention as user interface 18. For example, the present invention contemplates that user interface 18 may be integrated with a removable storage interface provided by electronic storage 16. In this example, information may be loaded into system 10 from removable storage (e.g., a smart card, a flash drive, a removable disk, etc.) that enables the user(s) to customize the implementation of system 10. Other exemplary input devices and techniques adapted for use with system 10 as user interface 18 include, but are not limited to, an RS-232 port, RF link, an IR link, modem (telephone, cable or other). In short, any technique for communicating information with system 10 is contemplated by the present invention as user interface 18.

One or more gas parameter sensors 20 are configured to generate one or more output signals conveying information related to one or more gas parameters of the gas breathed by subject 12. For example, the one or more gas parameter sensors 20 may be used to sense and convey information to determine the respiratory rate. The one or more gas parameter sensors 20 may also be configured to sense other parameters, such as, for example, one or more of a volume, a pressure, a composition (e.g., concentration(s) of one or more constituents), humidity, temperature, acceleration, velocity, acoustics, changes in a parameter indicative of respiration, and/or other gas parameters. In an embodiment in which a pressurized flow of breathable gas is delivered to subject 12 from device 14, sensors 20 include sensors in communication with gas within subject interface 24. System 10 may also include one or more optional additional physiological sensors 21 configured to sense physiological characteristics of subject 12. For example, sensors 21 may include a pulse oximeter configured to monitor the oxygen saturation of a patient's blood. Sensors 21 may also include a cardiac monitor to monitor, for example, subject 12's cardiac rhythm and/or heart rate variability. It should be appreciated that sensors 21 may also include other types of sensors and any combination and number thereof.

Processor 22 is configured to provide information processing capabilities in system 10. As such, processor 22 may include one or more of a digital processor, an analog processor, a digital circuit designed to process information, an analog circuit designed to process information, a state machine, and/or other mechanisms for electronically processing information. Although processor 22 is shown in FIG. 1 as a single entity, this is for illustrative purposes only. In some implementations, processor 22 may include a plurality of processing units. These processing units may be physically located within the same device, or processor 22 may represent processing functionality of a plurality of devices operating in coordination.

As is shown in FIG. 1, processor 22 may be configured to execute one or more computer program modules. The one or more computer program modules may include one or more of a secondary therapy mode module 32, a primary therapy mode module 34, a transition module 36, an interface module 38 and/or other modules. Processor 22 may be configured to execute modules 32, 34, 36, and/or 38 by software; hardware; firmware; some combination of software, hardware, and/or firmware; and/or other mechanisms for configuring processing capabilities on processor 22.

It should be appreciated that although modules 32, 34, 36, and/or 38 are illustrated in FIG. 1 as being co-located within a single processing unit, in implementations in which processor 22 includes multiple processing units, one or more of modules 32, 34, 36, and/or 38 may be located remotely from the other modules. The description of the functionality provided by the different modules 32, 34, 36, and/or 38 described below is for illustrative purposes, and is not intended to be limiting, as any of modules 32, 34, 36, and/or 38 may provide more or less functionality than is described. For example, one or more of modules 32, 34, 36, and/or 38 may be eliminated, and some or all of its functionality may be provided by other ones of modules 32, 34, 36, and/or 38. As another example, processor 22 may be configured to execute one or more additional modules that may perform some or all of the functionality attributed below to one of modules 32, 34, 36, and/or 38.

Secondary therapy mode module 32 is configured to provide the secondary therapy. In one embodiment, the secondary therapy includes breathing cues that are provided to subject 12 that encourage subject 12 to consciously adjust respiration such that the one or more breathing parameters are altered to conform to a breathing regime. The breathing parameters may also include one or more of an inhalation flow rate, an inhalation period, an exhalation flow rate, and/or an exhalation period. The breathing parameters may also include one or more of an inhalation flow rate, an inhalation period, an exhalation flow rate, and/or an exhalation period. In some embodiments, a timer may be configured to determine the duration of inhalation and exhalation and/or the time therebetween to determine the breathing rate. In some embodiments, the one or more breathing parameters may optionally include parameters of the actual gas breathed by subject 12 (e.g., one or more of a tidal volume, a breath period, a peak flow, a flow curve shape, a pressure curve shape, and/or other breathing parameters).

In one embodiment, secondary therapy mode module 32 may be configured to control device 14 to provide breathing cues based on a target for the one or more breathing parameters. For example, the target may be a target breathing rate. In one embodiment, if the breathing parameter is a curve shape, the target may include a target curve shape. In one embodiment, the target may be based at least partially on information received from sensors 20, 21.

In one embodiment, the target is received from a user (e.g., a caregiver, subject 12, etc.). The user may input the target via user interface 18. Inputting the target may include inputting a new target, or adjusting a previously obtained target. Inputting the target may also include configuring the target from a predetermined template (e.g., corresponding to a certain breathing regime). Subject 12 or other users may also input information via user interface 18 to adjust or change the order of the breathing patterns and the target associated with the breathing patterns.

The target for the one or more breathing parameters corresponds to a breathing regime. If the breathing regime includes a flow rate curve shape, the target may include a target curve shape. The target curve shape may be refined (e.g., by a user via user interface 18) to a target curve having values for the extrema (e.g., maxima and/or minima). Subject 12 or other users may input information via user interface 18 to adjust the target or input further information for the predictive algorithm to set the target. In one embodiment, subject 12 may input information via user interface 18 at any time during therapy. Other targets corresponding to these and/or other breathing regimes may be implemented within the scope of this disclosure.

In an embodiment in which device 14 generates the pressurized flow of breathable gas according to a BiPAP mode, secondary therapy mode module 32 may control device 14 to adjust the timing of the HI and LO segments. For example, when comparison module 34 determines that subject 12's breathing rate is greater than the target rate, secondary therapy mode module 32 may control device 14 such that the time over which the HI pressure of the device 14 is supplied is increased and the time over which the LO pressure is supplied is adjusted. The LO pressure time may be adjusted in accordance with a fixed ratio between the HI and Lo pressure times, or may be adjusted to coincide with subject 12's actual expiration time. Thus, by controlling the pattern or timing of the application of HI and LO pressures, the breathing rate of subject 12 may be effected. In other embodiments in which device 14 generates the pressurized flow of breathable gas according to a BiPAP mode, secondary therapy mode module 32 may control device 14 to adjust other properties of the HI and LO pressures, such as a period of the HI and/or LO pressure cycles, a pressure curve shape during a transition between HI and LO pressure cycles, a flow rate curve shape during a transition between HI and LO pressure cycles, and/or adjust other gas parameters of the pressurized flow of breathable gas. Such adjustments to the gas parameters of the pressurized flow of breathable gas will tend to provide breathing cues to subject 12 to consciously other breathing parameters in addition to or instead of breath rate. For example, such breathing cues may also prompt subject 12 to alter one or more of a respiration flow curve shape, a respiration pressure curve shape, and/or other breathing parameters.

It is contemplated that in some embodiments, secondary therapy mode module 32 may also control device 14 to adjust other breathing parameters of subject 12, such as the pressure, flow rate, and/or volume of gas delivered to the airway of subject 12 while the pressurized flow of breathable gas is being generated at the HI pressure (e.g, during inhalation) and/or at the LO pressure (e.g., during exhalation). Adjusting the pressure, flow rate, and/or volume of gas delivered to the airway of subject 12 while the pressurized flow of breathable gas is being generated at the HI pressure or at the LO pressure will tend to generate breathing cues that prompt subject 12 to alter the volume of gas inhaled/exhaled, to alter the inspiration/exhalation period, to alter the inspiration/expiration flow rate, to alter the tidal volume, and/or to otherwise consciously alter one or more other breathing parameters. Secondary therapy mode module 32 may deliver breathing cues in a feedback manner based comparison between the breathing parameter and the target threshold or in a non-feedback manner where the breathing cues are provided according to a predetermined target or pattern.

Interface module 38 may control user interface 18 to provide information relating to the breathing cues provided to subject 12. The information related to the breathing cues may include, for example, instructions to begin exhaling, to end exhaling, to begin inhaling, to end inhaling, to breathe faster, to breathe slower, to pause respiration, and/or to otherwise consciously alter one or more breathing parameters. Interface module 38 may also control user interface 18 to provide information relating to the type of therapy provided to subject 12. The information may be provided to subject 12 by user interface 18 in the form of auditory signals, visual signals, tactile signals, and/or other sensory signals. By way of non-limiting example, user interface 18 may include a radiation source capable of emitting light. The radiation source may include, for example, one or more of at least one LED, at least one light bulb, a display screen, and/or other sources. For example, the radiation source may be configured to emit light in a manner that conveys to subject 12 information related to the breathing cues being provided to subject 12 by the pressurized flow of breathable gas. For instance, the radiation source may emit light when the breathing cues are prompting subject 12 to inhale, and may stop emitting light, or emit light of a different color, when the breathing cues are prompting subject 12 to exhale. The intensity of the light emitted by the radiation source may convey to subject 12 the magnitude of the flow that the breathing cues are prompting subject 12 to generate during respiration.

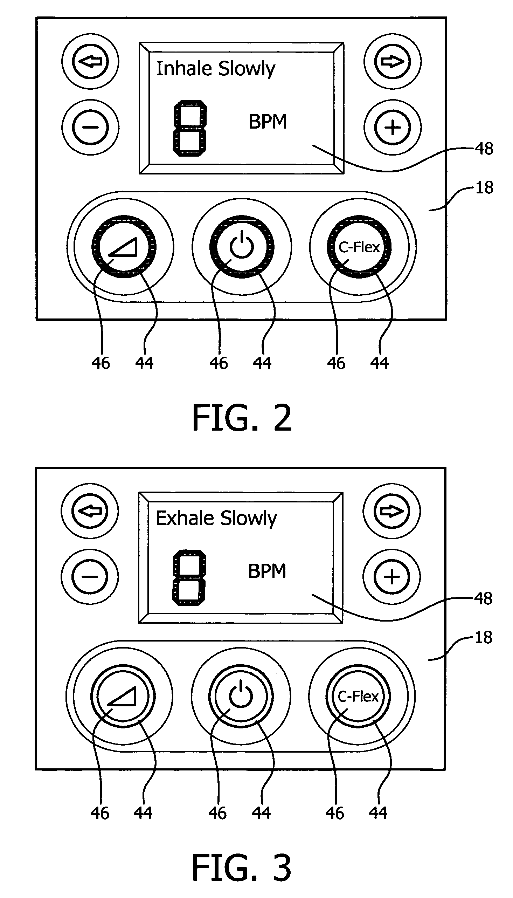

FIGS. 2 and 3 illustrate an embodiment in which a plurality of radiation sources 44 included in user interface 18 are configured to emit radiation in a manner that provides information about the breathing cues being delivered by the pressurized flow of breathable gas. In particular, the plurality of radiation sources 44 are integrated with a set of buttons 46 disposed on device 14 to control device 14. In the embodiment illustrated in FIGS. 2 and 3, radiation sources 44 emit radiation when the breathing cues are prompting subject 12 to inhale, and stop emitting radiation when the breathing cues are prompting subject 12 to exhale.

Alternatively or additionally, user interface 18 may include one or more elements capable of generating sounds that are audible to subject 12. Another alternative or additional way of communicating information may include one or more devices that contact subject 12 and provide tactile feedback to subject 12. For instance, user interface 18 may include a cuff that is worn by subject 12 around an extremity such as an arm, a leg, a finger, and/or other extremities. The cuff may carry one or more sensors configured to detect a physiological parameter of subject 12, such as for example, pulse, pulse rate, respiratory effort, blood pressure, blood oxygenation, and/or other physiological parameters. The cuff may vibrate and/or tighten on the extremity of subject 12 to provide information about the breathing cues or the type of therapy provided to subject 12, such as a transition between inhalation and/or exhalation, or that flow should be increased or decreased.

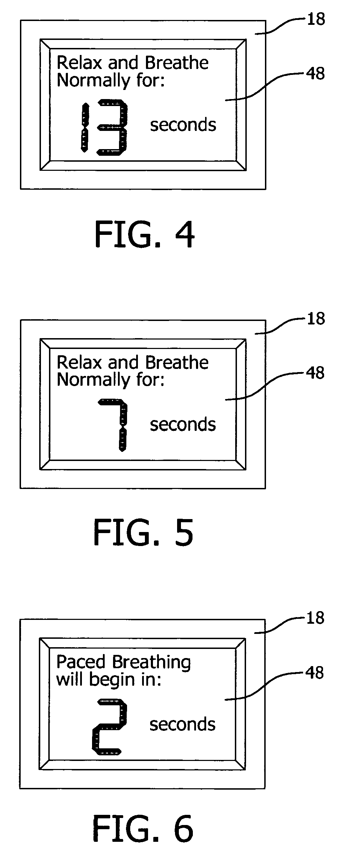

As another non-limiting example of the manner in which user interface 18 may communicate information about the breathing cues to subject 12, user interface 18 may include a display screen that provides subject 12 with text conveying information about the breathing cues. The display screen may include, for instance, a screen provided on device 14 and/or other display screens. For instance, FIGS. 2 and 3 illustrate user interface 18 including a display screen 48 for conveying information to subject 12 about the breathing cues being delivered by the pressurized flow of breathable gas. Figs. 4-6 illustrate other examples of display screen 48 configured to provide information to subject 12 regarding breathing cues.

Primary therapy mode module 34 is configured to control device 14 in accordance with the primary therapy wherein the flow of breathable gas is provided to the airway of subject 12 at a selected pressure or at selectively variable pressures. In embodiments where the primary therapy is provided in the form of CPAP, positive pressure may be applied at a substantially constant, patient-specific prescription pressure within the airway of subject 12 to maintain a "splint" force to sustain respiration. The level of the pressure may be determined based on a feedback manner, such as adjusting the pressure level and/or other gas parameters of the breathing bas supplied to subject 12 based on information received from sensors 20, 21. In one embodiment, the level of the pressure is automatically adjusted based on the detected conditions of the pressure, such as whether the patient is snoring or experiencing an apnea, hypopnea, or upper respiratory resistance. Alternatively, the pressure support may be provided in a non-feedback manner wherein the pressure level and/or other parameters of the breathing gas supplied to subject 12 is adjusted or set based on a predetermined level or pattern. The pattern or predetermined level may be set using user interface 18.

In embodiments where the primary therapy is provided in the form of BiPAP, pressure is applied alternatively at relatively higher and lower prescription pressure levels within the airway of subject 12 so that the pressure-induced patent force applied to subject 12's airway is alternatively a larger and a smaller magnitude force. The higher and lower magnitude positive prescription pressure levels (HI and LO, respectively) may be initiated by spontaneous subject respiration, pre-programming using user interface 18, or both, with the higher magnitude pressure (HI) applied during inspiration and the lower magnitude pressure (LO) being applied during expiration.

Primary therapy mode module 34 may control the pressure of the flow of breathing gas delivered to subject 12 by controlling the operating speed of device 14, either alone or in combination with the pressure controller 15. Primary therapy mode module 34 may also operate to control device 14 according to information inputted by the user via user interface 18. Furthermore, primary therapy mode module 34 may control user interface 18 to display information associated with the level of pressure provided to subject 12 and/or other types of information.

Transition module 36 is configured to provide a transition between the secondary therapy provided by secondary therapy mode module 32 and the primary therapy provided by primary therapy mode module 34 in response to a trigger event. Transition module 36 may be configured to detect the trigger event. The transition may include a gradual adjustment of one or more parameters of the breathing gas provided to subject 12. The trigger event may be associated with a time period, a sleep and/or awake state of subject 12, detection of sleep onset of subject 12, or any other events or status. For example, in one embodiment, the onset of sleep may be detected by an algorithm and the detection thereof may effect the completion of the secondary therapy, the gradual increase of pressure levels of the breathing gas provided to subject 12 until the pressure level reaches that of the primary therapy, and the commencement of the primary therapy. Alternatively or additionally, in one embodiment, transition module 36 may be configured to provide transition between the secondary and primary therapies based on manual input via user interface 18 or may be based on a preselected pattern or timing. For example, in one embodiment, user interface 18 may include a transition actuator constructed and arranged to be actuated to commence transition by transition module 36. The actuator may be constructed and arranged to provide visual and tactile feedback such that the user can reliably identify and operate the actuator either by sight or sense of touch.

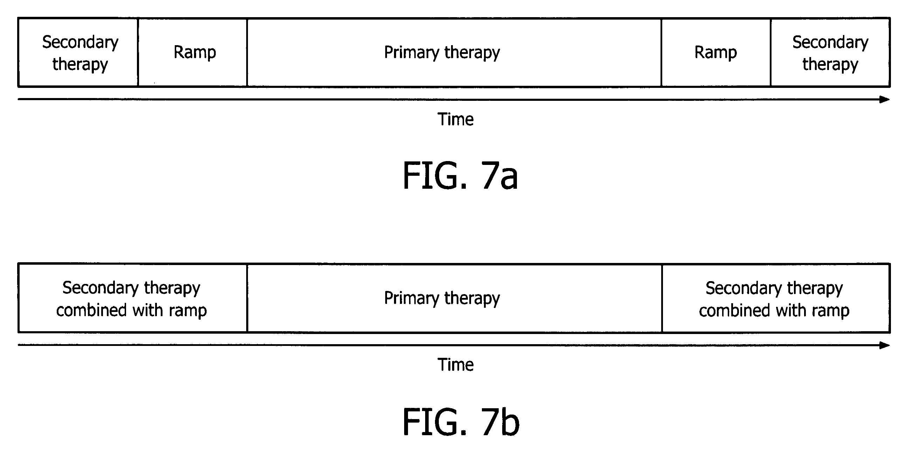

In one embodiment, transition module 36 is configured to control device 14 to adjust the pressure level during transition between the secondary therapy and the primary therapy. As mentioned above, a sudden change from the secondary therapy to the primary therapy may cause discomfort to subject 12 and may be distracting to subject 12 trying to fall asleep. Accordingly, transition module 36 may gradually adjust the pressure of the breathing gas provided to the airway of subject 12 such that the change between the primary therapy and the secondary therapy may be more comfortable to subject 12. For example, in one embodiment, transition module 36 may be configured to control device 14 to provide a transition wherein the pressure level and/or other gas parameters are adjusted during a time period between when the primary and secondary therapies are being supplied, as shown in Fig. 7a. In one embodiment, after the secondary therapy is completed and before the primary therapy is initiated, transition module 36 may control device 14 to increase the pressure of the breathing gas provided to subject 12 until the pressure reaches the level in accordance with the primary therapy. In such embodiment, transition module 36 may also decrease the pressure of the breathing gas provided to subject 12 during the change between the primary therapy and the secondary therapy. In other embodiments, transition module 36 may be configured to control device 14 to adjust the pressure level and/or other gas parameters during the secondary therapy, as shown in Fig. 7b. That is, in one embodiment, while secondary therapy is being provided to subject 12, transition module 32 may gradually adjust the pressure level of the breathing gas provided to subject 12 during secondary therapy. In such embodiment, secondary therapy mode module 32 may be configured to provide secondary therapy while transition module 36 gradually adjusts the pressure level of the breathing gas provided to subject 12 until the pressure level reaches the level associated with the primary therapy. It should be appreciated that transition module 36 may adjust other gas parameters of the breathing gas provided to subject 12 during the transition between the primary and secondary therapies in addition to or instead of pressure levels of the breathing gas. It should also be appreciated that transition module 36 may be configured to provide transition by adjusting the pressure level and/or other gas parameters at any time and for any duration during or between the secondary and primary therapies.

Transition module 36 may adjust the pressure level or other parameters of the breathing gas provided to subject 12 according to a predetermined pattern or shape during transition. For example, the pressure level or other parameters may be adjusted based on a linear, logarithmic, polynomial, power, exponential, moving average that is increasing or decreasing in pressure over time, or other functions. Other properties of the transition, such as rate or length, may also be controlled and/or adjusted by transition module 36. For example, the length of the transition may be a function of time, number of breaths, or other properties, including, but not limited to the breathing parameters of subject 12. The rate at which the pressure level or other gas parameters is adjusted may be a function of flow, leak, breath rate, breath ratio, minute ventilation, time, and/or other gas parameters and/or the breathing parameters. Accordingly, transition module 36 may be configured to adjust one or more gas parameters of the breathing gas according to a predetermined pattern or algorithm. Alternatively or additionally, transition module 36 may adjust one or more gas parameters of the breathing gas according to user input via user interface 18. For example, user interface 18 may be used to select the function for the adjustment of the pressure level, the length of the transition, and/or the rate at which the gas parameters is adjusted.

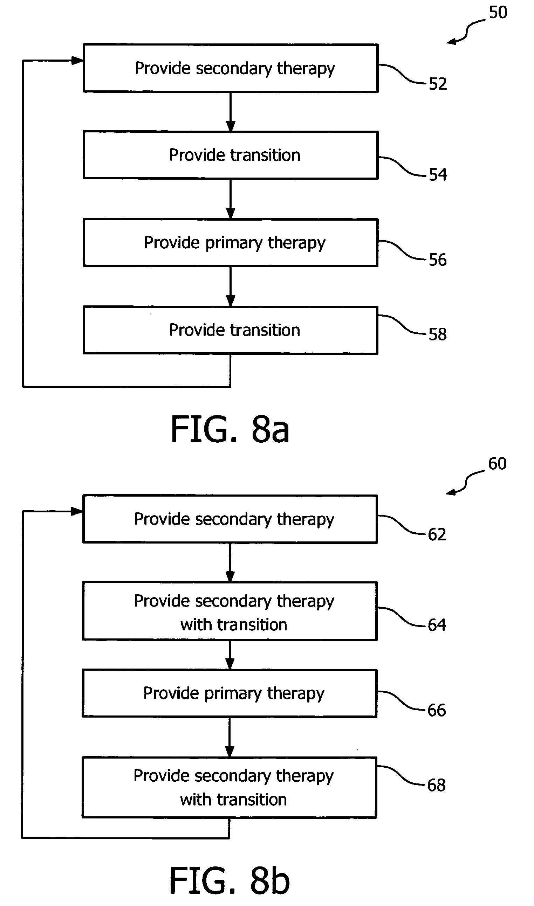

Fig. 8a illustrates a method 50 to provide pressure support to subject 12. The operations of method 50 are intended to be illustrative. In some embodiments, method 50 may be accomplished with one or more additional operations not described, and/or without one or more of the operations discussed. Additionally, the order in which the operations of method 50 are illustrated in Fig. 8 and described below is not intended to be limiting. In some embodiments, method 50 may be implemented in a system that is similar to or the same as system 10 (shown in Fig.1 and described above).

At operation 52, secondary therapy in the form of breathing cues are provided to subject 12. The breathing cues prompt subject 12 to breathe such that the breathing rate of subject 12 is in compliance with the target breathing rate that is based on a breathing regime. In one embodiment, the breathing cues include changes in one or more parameters of a pressurized flow of gas being delivered to the airway of subject 12. In one embodiment that uses BiPAP therapy, the changes include changes in patterns or timing of the applications of HI and LO pressures. In one embodiment, the breathing cues are provided by a device that is the same as or similar to device 14 (shown in Fig. 1 and described above). Device 14 may be controlled by a secondary therapy mode module that is the same as to similar to secondary therapy mode module 32 (shown in Fig. 1 and described above) to deliver the breathing cues to subject 12. Information relating to the breathing cues may also be provided to subject 12 via a user interface 18 that is the same as or similar to the user interface 18 (shown in Fig.1 and described above). A trigger event, such as a time period, an awake/sleep status of subject 12, detection of sleep onset of subject 12, a predetermined pattern, according to user input via user interface 18, or other events or statuses, causes method 50 to proceed to operation 54.

At operation 54, the secondary therapy provided by secondary therapy mode module 32 is completed and a transition is provided to subject 12 wherein the pressure level of the breathable gas provided to subject 12 is increased gradually according to a linear function or other functions. The rate of the transition is based on a function of time or any functions mentioned above. The length of the transition is based on a function of time or other functions mentioned above. The pressure level is gradually increased until the pressure level reaches the level associated with the primary therapy. The transition may be provided by a transition module that is the same as or similar to transition module 36 (shown in Fig. 1 and described above).

At operation 56, primary therapy is provided to the airway of subject 12 at a selected pressure or selectively variable pressures. In embodiments where the primary therapy is provided in the form of CPAP, positive pressure is applied at a substantially constant, patient-specific prescription pressure within the airway of subject 12. In embodiments where the primary therapy is provided in the form of BiPAP, pressure is applied alternatively at relatively higher and lower prescription pressure levels within the airway of subject 12 so that the pressure-induced patent force applied to subject 12's airway is alternatively a larger and a smaller magnitude force. Primary therapy may be provided by a primary therapy mode module the same as or similar to the primary therapy mode module 34 (shown in Fig.1 and described above). A trigger event, such as a time period, an awake/sleep status of subject 12, detection of sleep onset of subject 12, a predetermined pattern, according to user input via user interface 18, or other events or statuses, causes method 50 to proceed to operation 58.

At operation 58, primary therapy is completed and a transition is provided to subject 12 wherein the pressure level of the breathable gas provided to subject 12 is decreased gradually according to a linear function or other functions. The transition may be opposite that of the transition provided in operation 54. The pressure level is gradually decreased until the pressure level reaches the level associated with the secondary therapy, at which time the transition may be completed. The method 50 may then proceed back to operation 52 wherein the secondary therapy is provided to subject 12.

Fig. 8b illustrates another method 60 to provide pressure support to subject 12. The operations of method 60 are intended to be illustrative. In some embodiments, method 60 may be accomplished with one or more additional operations not described, and/or without one or more of the operations discussed. Additionally, the order in which the operations of method 60 are illustrated in Fig. 8b and described below is not intended to be limiting. In some examples, method 60 may be implemented in a system that is similar to or the same as system 10 (shown in Fig.1 and described above).

At operation 62, secondary therapy in the form of breathing cues are provided to subject 12. The breathing cues prompt subject 12 to breathe such that the breathing rate of subject 12 is in compliance with the target breathing rate that is based on a breathing regime. In one embodiment, the breathing cues include changes in one or more parameters of a pressurized flow of gas being delivered to the airway of subject 12. Device 14 may be controlled by a secondary therapy mode module that is the same as to similar to secondary therapy mode module 32 (shown in Fig. 1 and described above) to deliver the breathing cues to subject 12. Information relating to the breathing cues may also be provided to subject 12 via a user interface 18 that is the same as or similar to user interface 18 (shown in Fig.1 and described above). A trigger event, such as a time period, an awake/sleep status of subject 12, detection of sleep onset of subject 12, a predetermined pattern, according to user input via user interface 18, or other events or statuses, causes method 60 to proceed to operation 64.

At operation 64, the secondary therapy is continued to be provided to subject 12. In addition, a transition is provided wherein the pressure level of the breathing gas 12 provided to subject 12 during the secondary therapy is gradually increased until the level reaches the level associated with the primary therapy. The transition may be provided by a transition module that is the same as or similar to transition module 36 (shown in Fig. 1 and described above). After the pressure level has reached the level associated with the primary therapy, method 60 proceeds to operation 66.

At operation 66, primary therapy is provided to the airway of subject 12 at a selected pressure or selectively variable pressures. Primary therapy may be provided by a primary therapy mode module the same as or similar to the primary therapy mode module 34 (shown in Fig.1 and described above). A trigger event, such as a time period, an awake/sleep status of subject 12, detection of sleep onset of subject 12, a predetermined pattern, according to user input via user interface 18, or other events or statuses, causes method 60 to proceed to operation 68.

At operation 68, primary therapy is completed and a transition is provided to subject 12 wherein the pressure level of the breathable gas provided to subject 12 is decreased gradually according to a linear function or any other functions. In this operation 58, the secondary therapy in the form of breathing cues is also provided to subject 12 but at a pressure level determined by transition module 36. The transition may be opposite that of the transition provided in operation 64. Accordingly, the pressure level is gradually decreased until the pressure level reaches the level associated with the secondary therapy, at which time the transition may be completed. Method 60 may then proceed back to operation 62 wherein the secondary therapy is provided to subject 12 at a pressure level associated with the secondary therapy.

It should be appreciated that other modes of therapies may be provided and any combination of the therapies may be provided in other embodiments. It should also be appreciated that transition module 36 may be configured to provide transition between or during any combination of the various modes of therapies.

In the claims, any reference signs placed between parentheses shall not be construed as limiting the claim.

## Claims

1. A pressure support system (10) comprising:
a pressure generator (14) configured to generate a pressurized flow of breathable gas for delivery to the airway of a subject (12);
a processor (22) configured to execute computer program modules, the computer program modules comprising:
a first mode module (32) configured to control the pressure generator in accordance with a first mode of therapy wherein breathing cues are provided to the subject in accordance with a breathing regime using the pressurized flow of breathable gas, wherein the breathing cues prompt the subject to consciously alter one or more breathing parameters of respiration; and
a second mode module (34) configured to control the pressure generator in accordance with a second mode of therapy wherein the flow of breathable gas is provided to the airway of the subject at a selected pressure or selectively variable pressures;
a transition module (36) configured to transition between the first mode of therapy and a second mode of therapy wherein a pressure level of the gas in the pressurized flow of breathable gas is adjusted in response to a trigger event
**characterized in that** the transition provided by the transition module (36) between the first mode of therapy and the second mode of therapy includes a gradual adjustment of the pressure level of the gas in the pressurized flow of breathable gas.

2. The pressure support system of claim 1, wherein the trigger event comprises a time period.

3. The pressure support system of claim 1, wherein the trigger event comprises a sleep and/or awake state of the subject.

4. The pressure support system of claim 1, wherein the first mode module is configured to control the pressure generator to provide the transition during the first mode of therapy.

5. The pressure support system of claim 1, wherein the pressure generator is configured to provide the transition separately from the first mode of therapy and the second mode of therapy.

## Patentansprüche

1. Druckunterstützungssystem (10), das Folgendes umfasst:
einen Druckgenerator (14), der konfiguriert ist, um eine unter Druck stehende Atemgasströmung zur Abgabe an die Atemwege eines Patienten (12) zu erzeugen;
einen Prozessor (22), der konfiguriert ist, um Computerprogrammmodule auszuführen, wobei die Computerprogrammmodule Folgendes umfassen:
ein erstes Modusmodul (32), das konfiguriert ist, um den Druckgenerator in Übereinstimmung mit einem ersten Therapiemodus zu steuern, wobei dem Patienten Atemhinweise in Übereinstimmung mit einem Atemregime unter Verwendung des unter Druck stehenden Atemgases gegeben werden, wobei die Atemhinweise den Patienten auffordern, einen oder mehrere Atemparameter der Atmung bewusst zu ändern; und
ein zweites Modusmodul (34), das konfiguriert ist, um den Druckgenerator in Übereinstimmung mit einem zweiten Therapiemodus zu steuern, wobei die Atemgasströmung den Atemwegen des Patienten mit einem ausgewählten Druck oder selektiv variablen Druckwerten bereitgestellt wird;
ein Übergangsmodul (36), das konfiguriert ist, um einen Übergang zwischen dem ersten Therapiemodus und einem zweiten Therapiemodus zu schaffen, wobei ein Druckpegel des Gases in der unter Druck stehenden Atemgasströmung in Reaktion auf ein auslösendes Ereignis angepasst wird,
**dadurch gekennzeichnet, dass** der durch das Übergangsmodul (36) geschaffene Übergang zwischen dem ersten Therapiemodus und dem zweiten Therapiemodus eine allmähliche Anpassung des Druckpegels des Gases in der unter Druck stehenden Atemgasströmung umfasst.

2. Druckunterstützungssystem nach Anspruch 1, wobei das auslösende Ereignis eine Zeitspanne umfasst.

3. Druckunterstützungssystem nach Anspruch 1, wobei das auslösende Ereignis einen Schlaf- und/oder Wachzustand des Patienten umfasst.

4. Druckunterstützungssystem nach Anspruch 1, wobei das erste Modusmodul konfiguriert ist, um den Druckgenerator so zu steuern, dass er den Übergang während des ersten Therapiemodus schafft.

5. Druckunterstützungssystem nach Anspruch 1, wobei der Druckgenerator konfiguriert ist, um den Übergang getrennt von dem ersten Therapiemodus und dem zweiten Therapiemodus zu schaffen.

## Revendications

1. Système de support de pression (10) comprenant :
un générateur de pression (14) configuré pour générer un écoulement sous pression de gaz respirable pour un acheminement jusqu'aux voies respiratoires d'une personne (12) ;
un processeur (22) configuré pour exécuter des modules de programme informatique, les modules de programme informatique comprenant :
un premier module de mode (32) configuré pour commander le générateur de pression conformément à un premier mode de thérapie dans lequel des signaux respiratoires sont apportés à la personne conformément à un régime respiratoire en utilisant l'écoulement sous pression de gaz respirable, dans lequel les signaux respiratoires incitent la personne à modifier consciemment un ou plusieurs paramètres respiratoires de la respiration ; et
un deuxième module de mode (34) configuré pour commander le générateur de pression conformément à un deuxième mode de thérapie dans lequel l'écoulement de gaz respirable est apporté aux voies respiratoires de la personne à une pression sélectionnée ou à des pressions sélectivement variables ;
un module de transition (36) configuré pour passer du premier mode de thérapie au deuxième mode de thérapie dans lequel un niveau de pression du gaz dans l'écoulement sous pression de gaz respirable est ajusté en réponse à un événement déclencheur
**caractérisé en ce que** la transition assurée par le module de transition (36) entre le premier mode de thérapie et le deuxième mode de thérapie inclut un ajustement progressif du niveau de pression du gaz dans l'écoulement sous pression de gaz respirable.

2. Système de support de pression selon la revendication 1, dans lequel l'événement déclencheur comprend une période de temps.

3. Système de support de pression selon la revendication 1, dans lequel l'événement déclencheur comprend un état de sommeil et/ou un état éveillé de la personne.

4. Système de support de pression selon la revendication 1, dans lequel le premier module de mode est configuré pour commander le générateur de pression afin d'assurer la transition pendant le premier mode de thérapie.

5. Système de support de pression selon la revendication 1, dans lequel le générateur de pression est configuré pour assurer la transition séparément du premier mode de thérapie et du deuxième mode de thérapie.
